# EUROPEAN PATENT SPECIFICATION

(11) **EP 0 528 903 B1**
(45) Date of publication and mention of the grant of the patent: **11.09.1996**
(21) Application number: 91909315.3
(22) Date of filing: 22.04.1991
(51) Int. Cl.: C12N 15/11, A61K 31/70

(54) **METHODS OF PREVENTING VIRAL REPLICATION**
METHODEN ZUR VERHINDERUNG VON VIRALEN REPLIKATIONEN
PROCEDES SERVANT A PREVENIR LA REPLICATION VIRALE

(30) Priority: 20.04.1990 US 511428
(43) Date of publication of application: 03.03.1993
(73) Proprietor: THE GENERAL HOSPITAL CORPORATION, Boston, MA 02114 (US)
(72) Inventor: BLUM, Hubert, E., W-7930 Ehingen (DE); LIANG, Tsanyang, Brookline, MA 02146 (US); GALUN, Eithan, Brookline, MA 02146 (US); WANDS, Jack, R., Waban, MA 02168 (US)
(74) Representative: Wright, Simon Mark
(86) International application number: US9102793
(87) International publication number: WO9116420

(56) References cited:
- WO-A-86/05516
- WO-A-90/02176
- JOURNAL OF VIROLOGY vol. 61, no. 12, December 1987, pages 3946 - 3949 INOKUCHI AND HIRASHIMA 'Interference with viral infection by defective RNA replicase'
- VIROLOGY vol. 160, 1987, pages 323 - 329 GENDELMAN ET AL. 'Molecular characterization of a polymerase mutant human immunodeficiency virus'
- NATURE. vol. 315, 13 June 1985, LONDON GB pages 601 - 603 COLEMAN ET AL. 'A novel immune system against bacteriophage infection using complementary RNA (micRNA)'
- LIVER vol. 8, 1988, pages 307 - 316 BLUM ET AL. 'Latent hepatitis B virus infection with full-length viral genome in a patient serologically immune to hepatitis B infection'
- Cell, Volume 56, issued 13 January 1989, SCHLICHT et al., "Synthesis and Encapsidation of Duck Hepatitis B Virus reverse Trans Criptase do not require formation of Core-Polymerase Fusion Proteins", pages 85-92
- Journal of Virology, Volume 63, Number 2, issued February 1989, TRAKTMAN et al., "Molecular Genetic Analysis of Vaccinia Virus DNA Polymerase Mutants", pages 841-846

## Description

### Field of the Invention

The invention relates to the prevention and treatment of viral diseases.

### Background of the Invention

The consequences of a viral infection depend upon a number of factors, both viral and host. These factors which affect pathogenesis include the number of infecting viral particles and their path to susceptible cells, the speed of viral multiplication and spread, the effect of the virus on cell functions, the host's secondary responses to the cellular injury, and the immunologic and non-specific defenses of the host. In general, the effects of viral infection include acute clinical diseases, asymptomatic infections, induction of various cancers, and chronic progressive neurological disorders. Viruses are potent infectious pathogenic agents because virions produced in one cell can invade other cells and thus cause a spreading infection. Viruses cause important functional alterations of the invaded cells, often resulting in their death.

Viral infections continue to be major medical problems throughout the world. For example, acute and chronic hepatitis virus infection and its sequelae present a major problem. In fact, there are probably 400 million people in the world today infected with hepatitis B virus. In this setting, there is a high risk of acute and fulminant hepatitis and chronic liver disease, including cirrhosis, chronic active hepatitis and the eventual development of hepatocellular carcinoma in individuals who remain chronic carriers of the virus.

The dramatic effects of the human immunodeficiency virus (HIV) provides another illustration of the results of viral disease. There are currently more than 27,700 diagnosed cases of AIDS in the United States and the U.S. Public Health Service predicts that by the end of 1991 more than 179,000 persons will have the disease. Thus, intense medical research is going to the development of diagnostic tools and vaccines to HIV. AIDS and hepatitis represent only two of the diseases wrought by viral infection.

Currently, methods are needed to stop viral replication and prevent the spread of the virus to additional cells. However, this goal presents considerable difficulties. A major problem is the problem of inhibiting the virus without harming the host cells. The dependence of viral multiplication on cellular genes limits the points of differential attack. Even the largest viruses have fewer biochemical reactions that are unique in relation to the cells of the host. Further, it is only after extensive viral multiplication and cellular alteration have occurred that viral infections become evident. Therefore, the most general approach to control is prophylaxis. Therapy in most cases is limited to situations where the killing of some uninfected cells can be tolerated if the damage is subsequently repaired.

Another important limitation of anti-viral therapy is the emergence of resistant mutants. In order to avoid their selection, the principles valid for bacteria are equally applicable to viruses: adequate dosage, multi-drug treatment, and avoiding therapy unless clearly indicated. Therefore, because of the serious nature of viral infection and the obstacles presented by the nature of the infecting virus, there is an urgent need for methods which control viral replication. A method which would be applicable to RNA and DNA viruses would have widespread applicability.

### DESCRIPTION OF RELATED LITERATURE

Replication strategy of the hepatitis B virus can be found in: Seeger et al., Science 232:477-484 (1986); Khudyakov et al., FEBS Letters 243:115-l18 (1989); Will et al., J. of Virol. 61:904-911 (1987); and Hirsch et al., Nature 344:552-555 (1990).

Retroviruses and retroviral integration is discussed in: Varmus, H., Science 240:1427-1435 (1988); and Grandgenett et al., Cell 60:3-4 (1990).

### BRIEF DESCRIPTION OF THE DRAWINGS

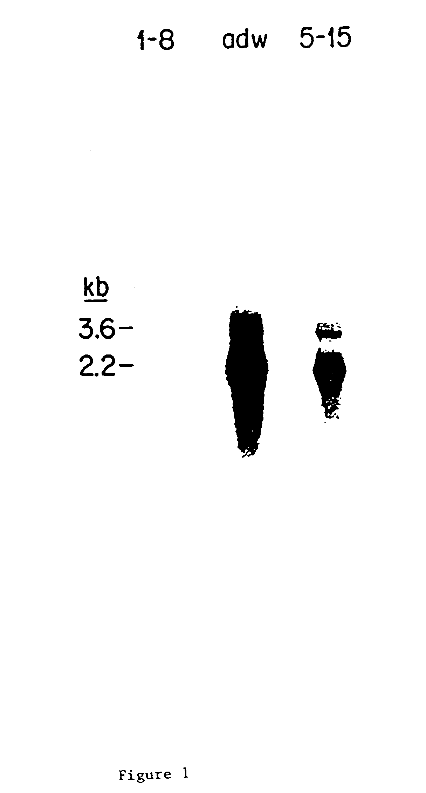

### Figure 1: Mutant HBV genome 5-15 is efficiently transcribed in Huh 7 hepatoma cells.

Huh 7 cells (Nakabayashi, H. et al., Cancer Res. 42:3858-3863 (1982)) were grown in MEM medium supplemented with 10% FBS. At about 90% confluence the cells were transfected with 20 ug cloned DNA per 100 mm dish, using the calcium phosphate method (Chen, C. et al., Mol. Cell. Biol. 7:2745-2752 (1987)). HBV DNAs used were the incomplete genome 1-8 (negative control; see Table I), a head-to-tail dimer of 'wild-type' adw and a head-to-tail dimer of the mutant viral genome 5-15. Forty-eight hours after transfection, total RNA was prepared from cells by the guanidine isothiocyanate method (Sambrook, J. et al., Molecular cloning: A laboratory manual, 2nd Edition, Cold Spring Harbor, NY, Cold Spring Harbor Laboratory Press (1989)), fractionated by formaldehyde 1.25% agarose gel electrophoresis, transferred to a Nytran membrane (Schleicher & Schuell, Keene, NH) and hybridized with a full-length HBV DNA probe, 32P-labeled by nick translation. Autoradiographic exposure at -80°C was 12 hours.

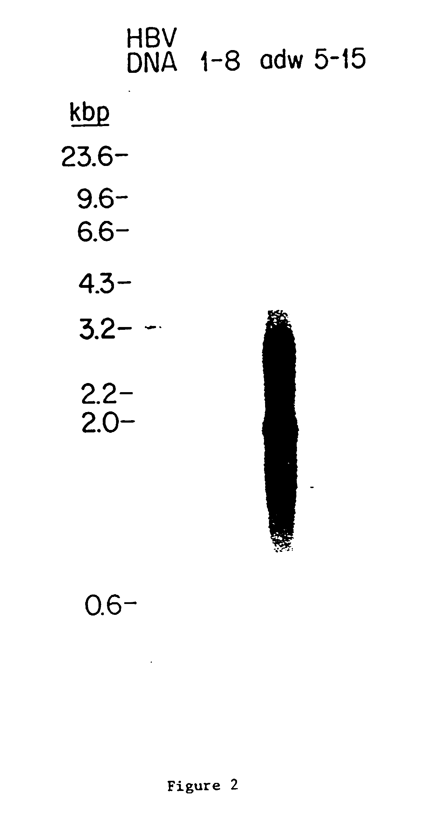

### Figure 2: Mutant HBV genome 5-15 is replication-defective.

Huh 7 cells were grown and transfected as described in the legend to figure 1. Five days after transfection cell culture media were collected and cells were lysed in NP40 (Hirsch, R. et al., Virology 167:136-142 (1988)). The cell lysate was centrifuged for 30 minutes at 50,000 x g (20°C). To the supernatant DNase I (1 ug per ml; Worthington) was added in order to digest input DNA. The solution was layered on a sucrose cushion (30% sucrose, 20 mM Tris-HCl, pH 7.4, 100 mM NaCl, 0.1% 2-mercaptoethanol, 0.1% bovine serum albumin) and centrifuged for 12 hours at 178,000 x g (4°C). The pellet was digested for 3 hours at 55°C with proteinase K (500 ug/ml) in the presence of 20 mM Tris-HCl, pH 8, 10 mM EDTA, 1% SDS. After phenol extraction, nucleic acids were precipitated with etnanol. After lyophilization, the precipitate was dissolved in 10 mM Tris-HCl, pH 7.4, 1 mM EDTA, and 1 ug DNase-free RNase A per ml, fractionated by 1.25% agarose gel electrophoresis, transferred to a Nytran membrane and hybridized to a full-length HBV DNA probe, 32P-labeled by nick translation. Cytoplasmic DNA from one 100 mm dish was applied per lane. Autoradiographic exposure at -80°C was 4 hours.

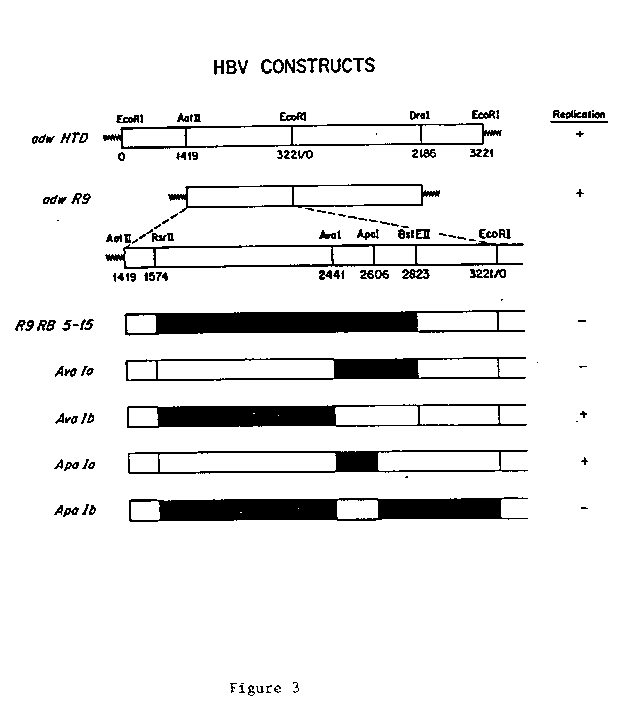

### Figure 3: DNA sequences responsible for replication-defectiveness of HBV mutant 5-15 map between nucleotide position 2606 (Apa 1) and 2823 (Bst E1I).

The head-to-tail dimer of 'wild-type' adw (adw HTD, cloned into the Eco RI site of pGEM-7) was subcloned as Aat Il (1419) and Dra I (2186) fragment to yield the truncated, replication-competent construct adw R9. By exchange of specific regions between adw R9 and the mutant HBV genome 5-15 the subclones illustrated were obtained. The regions in black indicate HBV 5-15 insertions into adw R9. The replicationcompetence of these constructs was analyzed as described in the legend to figure 2.

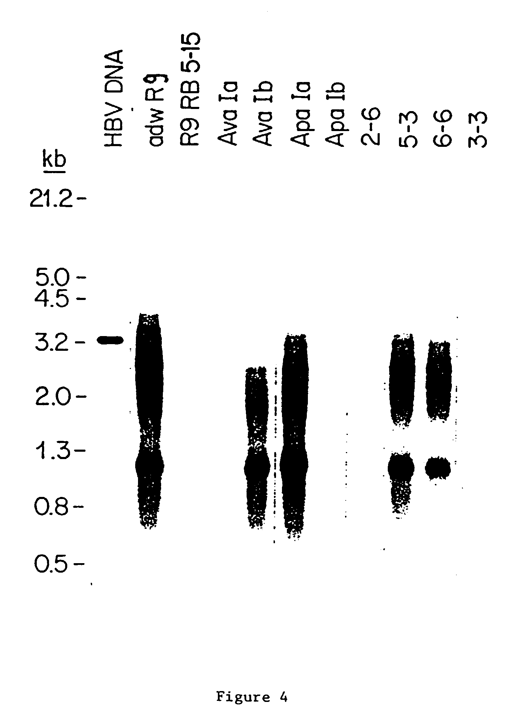

### Figure 4: Nucleotide 2798 is critical to replication competence of HBV DNA

Huh 7 cells were grown and transfected as described in the legend to figure 1. Five days after infection, cell culture media were collected and cells were lysed in NP40 (Hirsch et al., Virology 167:136-142 (1988)). Media were centrifuged for 30 minutes at 50,000 x g (20°C). The supernatant was brought to 10 mM MgCl₂. After addition of DNase I (1 ug/ml; Worthington) in order to digest input DNA, the solution was layered onto a sucrose cushion and processed as described in the legend to figure 2. The equivalent to the cell culture medium from one 100 mm dish (10 ml) was applied per lane. Autoradiographic exposure at -80°C was 12 hours. All cell culture media negative for replicating HBV DNA were also negative in the respective cell lysate preparations (data not shown). The structure and DNA sequence of the constructs, respectively, are given in Fig. 3 and Table 2, respectively.

### DESCRIPTION OF THE SPECIFIC EMBODIMENTS

Methods and compositions are provided for the termination of hepatitis viral replication. The methods involve providing the host cell with anti-sense RNA (aRNA).

While the intimate details of viral replication vary from virus to virus, certain aspects of the mechanism are shared. For example, RNA-directed DNA synthesis, first described for retroviruses, is now recognised as the probable means for the transfer of genetic information in various other settings. This includes the replication of hepatitis and cauliflower mosaic viruses. The synthesis of double-stranded DNA from a single-stranded RNA template requires an enzyme for synthesising the first DNA strand from an RNA template and a second DNA strand from the first; primers for each of the two strands; and a means for removing the RNA template after reverse transcription, to allow synthesis of the second DNA strand.

For purposes of the present invention, attention is focused on the role of the polymerase gene products in viral replication. The introduction of anti-sense RNA which is at least partially complementary to a portion of a polymerase gene of the hepatitis viral genome can effectively terminate replication of the virus. The methods are therefore applicable to hepatitis viruses which use polymerase gene products to prime transcription for RNA and DNA polymerases.

Nucleotide sequences of several viral genomes are published. For example, the nucleotide sequences of the HIV can be found in Ratner et al, Nature 313: 277-284 (1985). The nucleotide sequence of the hepatitis virus can be found in Okamoto et al, J. Gen. Virol. 69: 2575-2583 (1988) and available through Genbank.

Viral replication can be assayed by determining the presence of replication products or viral transcripts. In this manner, cell lysates and cell culture media can be analysed for the presence of viral transcripts, proteins, and replicative forms of viral DNA. To detect virus-specific transcripts, viral-encoded proteins may be identified by solid-phase radioimmunoassay, both in cell lysates and culture media. Specifically, the replication competence of a mutant viral genome may be assessed by Southern blot hybridisation of cytoplasmic DNA after transfection of cells with the mutant virus. See experimental section for more details. Also, details on hybridisation can be found in Nucleic Acid Hybridisation, A Practical Approach, IRL Press, Washington, DC (1985).

The expression of the mutant viral DNA in eukaryotic hosts requires the use of eukaryotic regulatory regions. Such regions will, in general, include a promoter region sufficient to direct the initiation of RNA synthesis. Typical promoters include the promoter of the mouse metallothionine I gene (Hammer, D. et al., J. Mol. Appl. Gen. 1:273-288 (1982)); the Tk promoter of herpes virus (McKnight, S., Cell 31:355-365 (1982)); the SV40 early promoter (Benoist et al., Nature 290:304-310 (1981)); and the like. Other useful promoters include liver specific promoters such as albumin, alpha-fetoprotein, alpha-1-antitrypsin, retinol-binding protein and others (Fang, X.J. et al., Hepatology 10:781-787 (1989)).

The desired mutant viral DNA and an operably linked promoter may be introduced into a recipient cell either as a non-replicating DNA or RNA molecule, which either may be a linear molecule or, more preferably, a closed covalent circular molecule. Since such molecules are incapable of autonomous replication, the expression of the desired receptor molecule may occur through the transient expression of the introduced sequence. Alternatively, where desired, permanent expression may occur through the integration of the introduced sequence into the host chromosome.

The introduced sequence may be incorporated into a plasmid or viral vector capable of autonomous replication in the recipient host. cDNA expression vectors include those described by Okayama, H., Mol. Cell. Biol. 3:280 (1983), and others. Viral vectors include retrovirus vectors as taught in W089/07136 (specifically for expression in hepatocytes) and the references cited therein.

In this manner, the corresponding nucleic acid or a portion of it coding for the specific mutation is introduced into the cells of a host suffering from the viral disease.

Supplying the anti-sense RNA to the virus interferes with replication. Anti-sense regulation has been described by Rosenberg et al, Nature 313: 703-706 (1985); Preiss et al, Nature 313: 27-32 (1985); Melton, Proc. Natl. Acad. Sci. USA 82:144-148 (1985); Kim et al., Cell 42:129-138 (1985); and Izant et al., Science 229:345-352 (1985). By supplying the anti-sense RNA the functioning of the naturally existing RNA is reduced. Thus, anti-sense regulation may be achieved by introducing into cells a DNA sequence comprising a gene in which the transcribed DNA sequences are at least partially complementary to the polymerase gene or gene region of interest of the virus. The introduced DNA will be under the transcriptional control of a transcriptional initiation region recognized by the host cells as discussed above. Transcription of the introduced DNA will result in multi-copies of an anti-sense RNA which will be complementary to RNA of the virus and result in reduction of functioning of the naturally existing RNA. Further, ribozymes spanning specific viral gene regions may serve as therapeutic agents (Sarver, N. et al., Science 247:1222-1225 (1990)).

It is also recognized that mutant viral genomes can be constructed and used to transfect and infect host cells. The mutant virus is capable of expressing the defective protein. Thus, the cells can effectively fight off viral attack. Where the host is already infected with unaltered virus, infection of the host with mutant virus expressing the defective protein leads to termination of replication of the unaltered virus.

The methods of the present invention can be utilized to prevent viral infection as well as to combat viral infections.

The compositions comprising the aRNA or DNA encoding aRNA RNA may be administered to prevent a virus infection or to combat the virus once it has entered the host.

As noted above, the compositions and methods of the invention can be used to terminate viral replication in hepatitis viruses.

The following examples are offered by way of illustration and not by way of limitation.

### EXPERIMENTAL

Episomal HBV DNA in the liver of an individual serologically immune to HBV infection was previously identified (Blum, H.E. et al., Liver 8:307-316 (1988)). The cloning, fine structure analysis, and biologic properties of the viral genome from this individual's livers has been accomplished. The cloned genome showed 48 base changes relative to an infectious HBV of the most closely related serotype adw2 (Valenzuela, P. et al., The nucleotide sequence of the hepatitis B viral genome and the identification of the major viral genes, p. 57-70, In B.N. Fields, R. Jaenisch and C.F. Fox (eds.), Animal virus genetics, Academic Press Inc., New York (1980)). One of these mutations introduced a stop codon at the end of the pre-core region of the viral genome, prohibiting hepatitis B e antigen (HBeAg) formation. Functional analysis of the cloned DNA by transfection into Huh 7 hepatoma cells indicated competence for synthesis of all major viral transcripts, viral core and envelope proteins but a defect blocking viral DNA replication. The genetic basis of this defect in DNA synthesis was identified to be a single missense mutation in position 2798 leading to a substitution of threonine or serine by proline. This finding suggests a critical role of this polymerase gene region, presumably coding for the terminal protein or a protein required for encapsidation of pregenomic RNA, in the life cycle of the virus as well as specific therapeutic strategies to terminate viral replication.

To establish the fine structure of the episomal HBV DNA in the patient's liver, the viral genome was amplified from total liver DNA by the polymerase chain reaction (PCR), using three HBV specific primer pairs (Table 1). The amplified viral DNA fragments were cloned in pGEM-7, yielding the three expected overlapping HBV clones 1-8, 2-13 and 3-1 (Table 1). Using these clones, the HBV DNA molecule was reconstructed and cloned in pGEM-7, yielding the full-length HBV genome 5-15. By direct sequencing of these clones, the complete nucleotide sequence of the HBV genome 5-15 was determined (data submitted to Genbank). The genome has a length of 3221 bases and a genetic organization identical to known HBV DNAs. A comparison to published DNA sequences demonstrated closest homology to HBsAg subtype adw2 (Valenzuela, P. et al., The nucleotide sequence of the hepatitis B viral genome and the identification of the major viral genes, p. 57-70, In B.N. Fields, R. Jaenisch and C.F. Fox (eds.), Animal virus genetics, Academic Press Inc., New York (1980)). As compared to this subtype, a total of 63 mutations was detected in HBV 5-15, 48 of which resulted in amino acid substitutions. To exclude mutations induced by PCR amplification, mutations leading to amino acid changes were confirmed by cloning and sequencing of independent PCR amplification products. Mutations were identified in all open reading frames with the highest frequency in the pre-C/ C region (26 per 1000 bases), a region which otherwise shows the highest degree of homology between different HBV DNAs and other hepadnaviruses (ref. 3 and data available through Genbank). While the mutations resulted in in-phase stop codon at the end of the pre-C reading frame, resulting in the inability to code for the pre-C/ C protein, a precursor to hepatitis B e antigen (HBeAg). The stop codon at the end of the pre-C reading frame was not found in the viral DNA integrated in the patient's hepatocellular carcinoma (data not shown), suggesting that this mutation was not present in the infecting virus but rather occurred during ongoing viral replication in the non-tumorous liver after integration of viral DNA.

Because no direct conclusions with respect to the biology of the virus could be drawn from the structural characteristics described above, the functional competence of the mutant viral genome was analyzed in vitro. Following transfection of Huh 7 hepatoma cells (Nakabayashi, H. et al., Cancer Res. 42:3858-3863 (1982)), cell lysates and cell culture media were analyzed for the presence of viral transcripts, proteins and replicative forms of viral DNA. As shown in Fig. 1, hybridization with an HBV-specific probe revealed the presence of two major transcripts of 2.2 and 3.6 kb length, respectively, in both head-to tail (HTD) adw ('wild-type') and HTD 5-15 ('mutant') transfected cells. No viral transcripts were present in non-transfected cells (data not shown) or in cells transfected with the incomplete HBV clone 1-8 (Table I). While the level of message was higher in HTD adw than in HTD 5-15 transfected cells, the ratio of the 3.6 kb pregenomic to 2.2 kb subgenomic message appears very similar. These findings indicate that the mutant viral genome is competent to synthesize all major HBV transcripts.

HBV-encoded proteins were identified by solid-phase radioimmunoassay, both in cell lysates and culture media. Both HTD adw and HTD 5-15 transfected Huh 7 cells secrete substantial amounts of HBsAg and HBc/eAg into the media while no viral antigens are produced by cells transfected with the incomplete clone HBV 1-8 (data not illustrated). As predicted from the stop codon mutation in position 1899, metabolic labeling and immunoprecipitation studies revealed that HTD 5-15 transfected cells are incompetent to synthesize the large pre-C/C protein and its smaller processed products, normally secreted as HBeAg into the cell culture media (data not illustrated).

In order to assess the replication competence of the mutant genome 5-15, HBV DNA species were analyzed by Southern blot hybridization of cytoplasmic DNA isolated from Huh 7 cells after transfection. As shown in Fig. 2, replicating HBV DNA species were detected in cells transfected with HTD adw only while no viral DNA was present in cells transfected with the incomplete clone HBV 1-8 (negative control) or with HTD 5-15. Viral DNA sequences in HTD adw transfected cells represent single-stranded and partially double-stranded replicative intermediates as well as complete relaxed circular molecules (Blum, H.E. et al., Virology 139:87-96 (1984)).

Viral antigens and DNA species in cell lysates and culture media of HTD adw or HTD 5-15 transfected cells were further characterized by CsCl density gradient centrifugation followed by analysis of individual fractions for HBsAg and HBc/eAg and HBV DNA sequences. In HTD adw transfected cells viral DNA is associated with HBc/eAg (core particles) and in culture media with HBsAg (virions) and HBc/eAg (core particles). As expected, while positive for HBsAg and HBcAg, no viral DNA species were detectable in CsCl fractions of cell lysates or culture media from HTD 5-15 transfected Huh 7 cells. These findings demonstrate that, while Huh 7 cells support viral replication, the mutant HBV genome 5-15 is unable to replicate in this system.

The molecular basis of the replication-defectiveness was determined by subcloning of specific regions of the mutant viral genome into the truncated replication-competent adw HTD construct adw R9 (Fig. 3). By exchange of specific DNA fragments, cloning and transfection of Huh 7 hepatoma cells, the mutations responsible for the replication-defectiveness could be localized to a region of the mutant viral genome mapping between position 2606 (Apa 1) and 2823 (Bst EII; Figs. 3 and 4). Within this region the mutant viral genome has only two missense mutations (nucleotide position 2798 and 2820) relative to adw2 (Valenzuela, P. et al., The nucleotide sequence of the hepatitis B viral genome and the identification of the major viral genes, p. 57-70, In B.N. Fields, R. Jaenisch and C.F. Fox (eds.), Animal virus genetics, Academic Press Inc., New York (1980)). By site-directed mutagenesis of either adw R9 or R9 RB 5-15 (Fig. 3) and primers carrying either the 2798 or the 2820 mutation four clones were generated by PCR amplification (Table 3): adw R9 with an A to C mutation in position 2798 (clone 2-6), adw R9 with a G to C mutation in position 2820 (clone 5-3), Ava Ia with a C to A mutation in position 2798 (clone 6-6), and Ava Ia with a C to G mutation in position 2820 (clone 3-3). After transfection of Huh 7 hepatoma cells with these clones, it could be demonstrated that the A to C mutation of clone adw R9 in position 2798 renders viral DNA replication-defective while the C to A mutation of clone Ava la in the same position reestablishes replicationcompetence (Fig. 4). By contrast, the mutation in position 2820 did not affect the replicationcompetence or -defectiveness of the parent constructs (clone 3-3 and clone 6-6). These findings unequivocally demonstrate that the A to C mutation in position 2798 of the viral genome, leading to a Thr to Pro substitution, is the molecular basis for the replication-defect of the mutant viral genome isolated from the patient's liver.

Experimental studies (Bosch, V. et al., Virology 166:474-485 (1988); Bartenschlager, R. et al., EMBO J. 7:4185-4192 (1988); Schlicht, H.-J. et al., Cell 56:85-92 (1989)) and comparative structural and hydropathy analyses of hepadnavirus polymerases (Khudyakov, Y.E. et al., FEBS Letters 243:115-118 (1989)) suggest that the 5' prime region of the viral polymerase gene encodes for the 'terminal protein' (TP). This protein binds to the 5' end of minus strand DNA and presumably serves as a primer of reverse transcription which is a central feature of the replication strategy of the hepadnaviruses (Seeger, C. et al., Science 232:477-484 (1986); Will, H. et al., J. Virol. 61:904-911 (1987)). Mutational analyses of the duck hepatitis B virus polymerase gene indeed demonstrated that an in-frame insertion into the 5' region of this gene eliminates the production of an active polymerase (Schlicht, H.-J. et al., Cell 56:85-92 (1989)). This data strongly suggests that the TP region of the polymerase gene is important for viral replication. Further, the functionally intact polymerase gene appears to be required for the packaging of pregenomic RNA. Consistent with this interpretation, the mutation identified in nucleotide position 2798 in our mutant or produced by mutagenesis of 'wild-type' virus terminates viral replication. The significance of this mutation is further emphasized by the fact that the amino acid in position 164 of the polymerase gene product is highly conserved in all HBV strains (11/14 Thr, 3/14 Ser), as shown by a comparison with the published HBV DNA sequences (Okamoto et al., J. Gen. Virol. 69:2575-2583 (1988)) and those available through the Genbank. The mechanism of this termination is possibly mediated through a conformational change of the TP or a protein involved in encapsidation of genomic RNA due to a Thr or Ser to a Pro substitution. Studies are in progress to define the contribution of this polymerase gene region to the function of these proteins.

Cotransfection of Huh 7 hepatoma cells with wild type HBV and mutant HBV DNA demonstrated that the mutant was capable of terminating replication of the wild type HBV. See Table 4. When HBV and unrelated DNA were used to transfect Huh 7 cells, replication of the hepatitis virus was not hindered as evidenced by hybridization of total cellular DNA with HBV viral DNA. No viral DNA was detected in cells cotransfected with mutant virus DNA, indicating that the mutant was able to terminate replication of the wild type hepatitis virus.

The capability of a mutant virus to terminate replication of nonmutant viruses has important implications. In those individuals or hosts suffering from a viral infection, the mutant virus can terminate the replication of the virus in infected cells and prevent the virus spreading to other cells.

All publications and patent applications mentioned in the specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

**TABLE 1:**

| Comparison of conservation of AA position 164 in the polymerase gene of various HBV strains | | | |
|---|---|---|---|
| | HBV Strain | AA-164 | NT. 2798 |
| 1. | vadw2 | Thr | ACC |
| 2. | adr | Thr | ACT |
| 3. | ayr | Thr | ACT |
| 4. | adra | Thr | ACT |
| 5. | adrm | Thr | ACT |
| 6. | adrcg | Thr | ACT |
| 7. | adw1 | Ser | TCA |
| 8. | adw2 | Ser | TCC |
| 9. | adw 3 | Thr | ACA |
| 10. | adw | Thr | ACC |
| 11. | vcg chimp | Thr | ACC |
| 12. | adyw | Ser | TCA |
| 13. | ayw | Thr | ACA |
| 14. | hpv | Thr | ACT |
| | | | |
| * Mutant (no replication) | | Pro | CCC |

| | | | |
|---|---|---|---|
| * A single mutation e.g. A to C at position 2798 converts Thr to Pro or a T to C mutation at position 2798 converts Ser to Pro. | | | |

### TABLE 2: Position and Sequences of HBV Specific Oligonucleotide Primers used for PCR amplification and Cloning of HBV DNA from the Patient's Liver

A 73-year old white male with a history of viral hepatitis presented with metastasizing hepatocellular carcinoma (HCC). HBV serology was negative for HBsAg and anti-HBc IgM and positive for anti-HBc IgG, anti-HBe and anti-HBs, as determined by radioimmunoassays (Abbott, North Chicago, IL). No HBV DNA could be detected in serum by spot-blot hybridization. Liver and HCC tissues obtained at autopsy were stored at -80°C until use. Liver DNA was amplified using Taq polymerase and primers carrying restriction enzyme sites at the 5' and 3' ends (Table 2). The polymerase chain reaction (PCR) was performed according to Saiki et al. (Saiki, R. et al., Science 239:487-4491 (1988)). Briefly, target sequences were amplified in a 50 ul reaction volume containing 50 ng DNA, 2.5 units of Taq polymerase (Perkin-Elmer Cetus), 200 uM each dNTP, 1 uM each primer, 50 ml/l KCl, 10 mM Tris-HCl, pH 8.3, 1.5 mM MgCl2, and 0.01% (wt/vol) gelatin. The reaction was performed for 40 cycles in a programmable DNA thermal cycler (Perkin-Elmer Cetus). Samples were heated to 94°C for 1.5 min (denaturation of DNA), cooled to 50°C for 1.5 min (hybridization to primer), and incubated for 3 min at 72°C (polymerase reaction). After PCR, 5 units of Klenow polymerase were added to complete strand synthesis and to ensure cleavage by the appropriate restriction endonucleases. The PCR mixture was fractionated on a 1.25% agarose gel in the presence of ethidium bromide. The band containing the target sequence was removed and DNA was isolated by glass beads (Geneclean, Bio 101 Inc., La Jolla, CA). The purified fragments were ligated into pGEM-7Zf(+) (Promega) and cloned in DH5αF' cells (BRL). Cloned DNA was prepared by standard plasmid preparation. The three clones 1-8, 2-13 and 3-1 (Table 2) were used to reconstruct the full-size viral genome (clone 5-15). For transfection experiments, head-to-tail dimers of HBV DNA HBsAg subtype adw ('wild-type') and of HBV DNA 5-15 were constructed in pGEM-7Zf(+) and cloned as described above. DNA sequence analysis was performed as described (Mierendorf, R.C. et al., Meth. Enzymol. 152:556-562 (1987)) using the Gem Seq RT system (Promega) or the T7 polymerase system (Pharmacia).

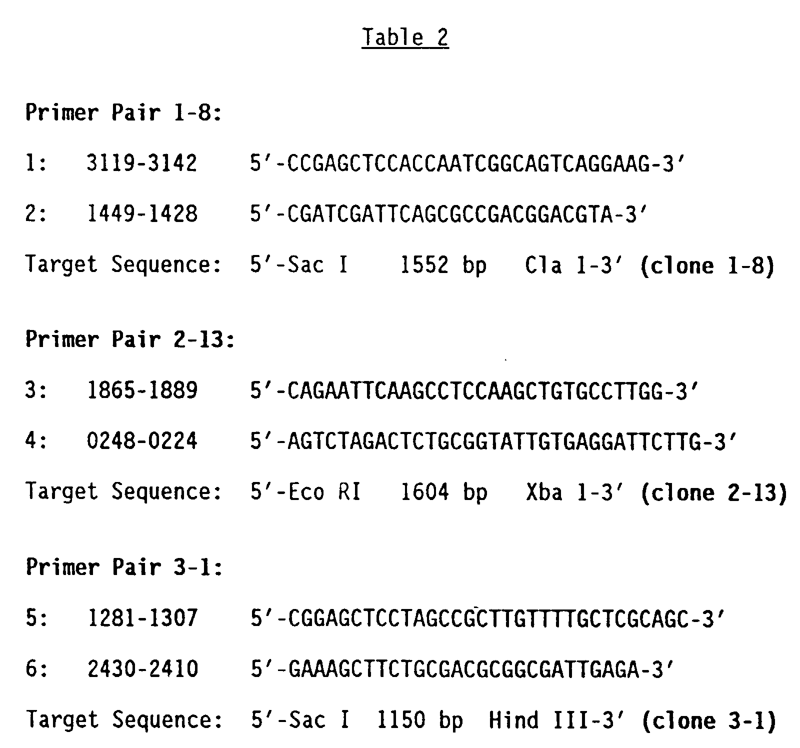

### TABLE 3: DNA Sequence of In Vitro Mutagenesized HBV DNA Clones and Their Parent Constructs

Clones adw R9 and R9 5-15 (see figure 3) were amplified by PCR (see Table 1) using a generic primer spanning the region 2312 to 2333 (CC-22) and a primer spanning the region 2833 to 2794, carrying either a T to G mutation in position 2798 (ATC-40) or a C to G mutation in position 2820 (ACC-40). The amplified fragments were purified by agarose gel electrophoresis and Geneclean (Bio 101 Inc., La Jolla, CA). After digestion with Apa I and Bst EII, the fragments were cloned into adw R9 (see figure 3), yielding the 4 clones 2-6, 3-3, 5-3 and 6-6 carrying the expected single point mutation.

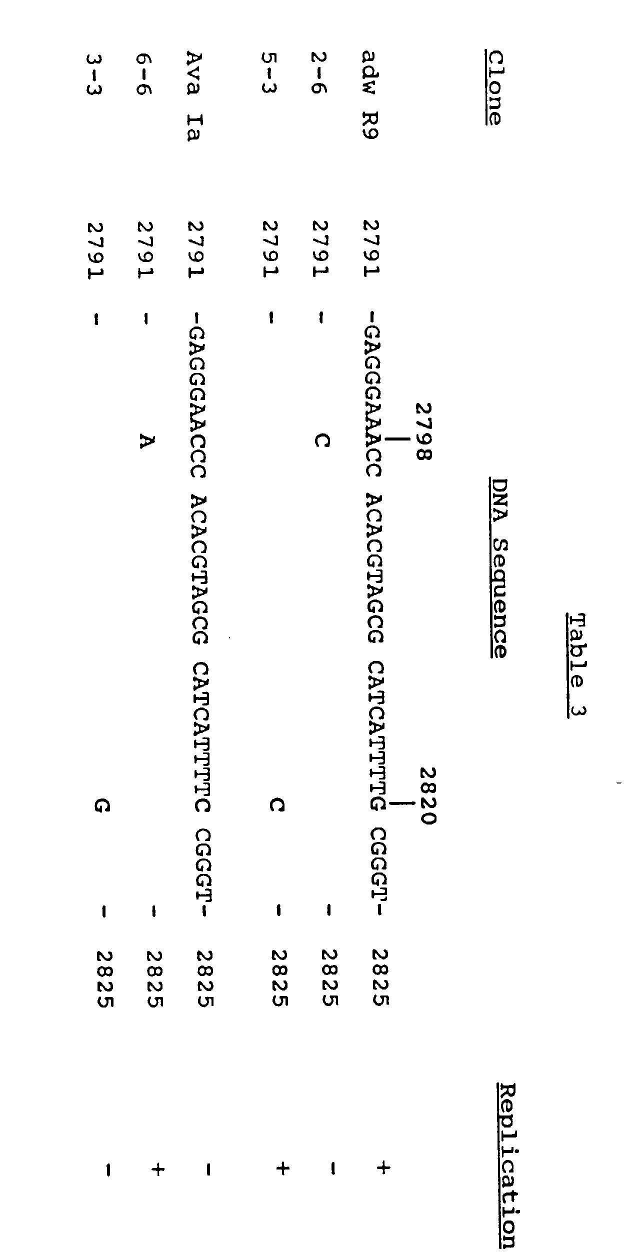

### TABLE 4: Cotransfection with mutant HBV

Huh 7 cells were cotransfected with: 1) wild type HBV and unrelated DNA; and, 2) wild type HBV and mutant HBV DNA. The unrelated DNA utilized for cotransfection was a neomycin resistance carrying vector.

After cotransfection, replication of the virus was determined by extraction of total cellular DNA followed by hybridization with labelled HBV DNA. Following extraction, total cellular DNA was digested with DNase and RNase followed by centrifugation in a sucrose mixture. Viral DNA was taken from the sucrose gradient and subjected to gel electrophoresis. Following electrophoresis the DNA was transferred to nitrocellulose and treated with labelled viral DNA. The resulting autoradiographs showed the presence of viral DNA in cells cotransfected with wild type HBV and unrelated DNA. No viral DNA was detected in the cells transfected with wild type HBV and mutant HBV DNA.

**Table 4**

| | Cotransfection | Replication |
|---|---|---|
| 1. | Wild type HBV and unrelated DNA | + |
| 2. | Wild type HBV and mutant HBV DNA | - |

| | | |
|---|---|---|
| + indicates replication of HBV as evidenced by hybridization with viral DNAs | | |
| - no replication detected | | |

## Claims (Claims for the following Contracting State(s): AT, BE, CH, DE, DK, FR, GB, IT, LI, LU, NL, SE)

1. An *in vitro* method of inhibiting hepatitis viral replication in a cell the method comprising introducing into the cell antisense RNA (aRNA) or DNA which is transcribed into the aRNA, the aRNA being at least partially complementary to a portion of a polymerase gene region of the hepatitis viral genome.

2. An *in vitro* method according to claim 1 wherein:
(a) the virus is hepatitis B virus;
(b) the DNA is regulated by a cell specific promoter; and/or
(c) the cell is a liver tissue cell.

3. Antisense RNA (aRNA), or a recombinant DNA molecule capable of being transcribed into an aRNA, the aRNA being at least partially complementary to mRNA encoding a hepatitis virus polymerase protein wherein the aRNA is capable of inhibiting hepatitis viral replication in a cell.

4. aRNA or DNA according to claim 3, wherein the hepatitis virus polymerase protein is from Hepatitis B virus.

5. A vector comprising aRNA or a DNA molecule according to claim 3 or claim 4.

6. aRNA or DNA according to claim 3 or claim 4; and/or a vector according to claim 5 for use in medicine.

7. The use of:
aRNA or DNA according to claim 3 or claim 4; and/or a vector according to claim 5
in the preparation of an agent for inhibiting or interrupting hepatitis viral replication, such as an agent for the prevention of hepatitis.

8. A composition adapted for administration to a patient comprising:
aRNA or DNA according to claim 3 or claim 4; and/or
a vector according to claim 5
and a suitable carrier.

## Claims (Claims for the following Contracting State(s): ES, GR)

1. An *in vitro* method of inhibiting hepatitis viral replication in a cell the method comprising introducing into the cell antisense RNA (aRNA) or DNA which is transcribed into the aRNA, the aRNA being at least partially complementary to a portion of a polymerase gene region of the hepatitis viral genome.

2. An *in vitro* method according to claim 1 wherein:
(a) the virus is hepatitis B virus;
(b) the DNA is regulated by a cell specific promoter; and/or
(c) the cell is a liver tissue cell.

3. An *in vitro* method according to claim 1 or 2 wherein the nucleic acid used is antisense RNA (aRNA), or a recombinant DNA molecule capable of being transcribed into an aRNA, the aRNA being at least partially complementary to mRNA encoding a hepatitis virus polymerase protein wherein the aRNA is capable of inhibiting viral replication in a cell.

4. The use of:
aRNA or DNA as defined in claim 3; and/or a vector comprising RNA or a DNA molecule as defined in claim 3;
in the preparation of an agent for inhibiting or interrupting hepatitis viral replication, such as an agent for the prevention of hepatitis.

## Patentansprüche (Patentansprüche für folgende(n) Vertragsstaat(en): AT, BE, CH, DE, DK, FR, GB, IT, LI, LU, NL, SE)

1. *In-vitro*-Verfahren zur Hemmung der Replikation von Hepatitis-Viren in einer Zelle, wobei das Verfahren die Einführung von Antisense-RNA (aRNA) oder DNA, die in die aRNA transkribiert wird, in die Zelle umfaßt, wobei die aRNA mindestens teilweise zu einem Teil einer Polymerasegenregion des Hepatitis-virus-Genoms komplementär ist.

2. *In-vitro*-Verfahren nach Anspruch 1, wobei
(a) das Virus das Hepatitis-B-Virus ist;
(b) die DNA von einem zellspezifischen Promotor reguliert wird; und/oder
(c) die Zelle eine Lebergewebezelle ist.

3. Antisense-RNA (aRNA) oder rekombinantes DNA-Molekül, das in eine aRNA transkribiert werden kann, wobei die aRNA mindestens teilweise zu mRNA komplementär ist, die für ein Hepatitis-Virus-Polymeraseprotein codiert, wobei die aRNA in der Lage ist, die Replikation von Hepatitis-Viren in einer Zelle zu hemmen.

4. aRNA oder DNA nach Anspruch 3, wobei das Hepatitis-Virus-Polymeraseprotein vom Hepatitis-B-Virus stammt.

5. Vektor, umfassend aRNA oder ein DNA-Molekül nach Anspruch 3 oder Anspruch 4.

6. aRNA oder DNA nach Anspruch 3 oder Anspruch 4; und oder Vektor nach Anspruch 5 zur Verwendung in der Medizin.

7. Verwendung von:
aRNA oder DNA nach Anspruch 3 oder Anspruch 4 und/oder einem Vektor nach Anspruch 5 bei der Herstellung eines Mittels zur Hemmung oder Unterbrechung der Replikation von Hepatitis-Viren, wie ein Mittel zur Verhütung von Hepatitis.

8. Zusammensetzung, vorgesehen zur Verabreichung an einen Patienten, umfassend:
aRNA oder DNA nach Anspruch 3 oder Anspruch 4; und/oder einen Vektor nach Anspruch 5 sowie einen geeigneten Träger.

## Patentansprüche (Patentansprüche für folgende(n) Vertragsstaat(en): ES, GR)

1. *In-vitro*-Verfahren zur Hemmung der Replikation von Hepatitis-Viren in einer Zelle, wobei das Verfahren die Einführung von Antisense-RNA (aRNA) oder DNA, die in die aRNA transkribiert wird, in die Zelle umfaSt, wobei die aRNA mindestens teilweise zu einem Teil einer Polymerasegenregion des Hepatitis-Virus-Genoms komplementär ist.

2. *In-vitro*-Verfahren nach Anspruch 1, wobei:
(a) das Virus das Hepatitis-B-Virus ist;
(b) die DNA von einem zellspezifischen Promotor reguliert wird; und/oder
(c) die Zelle eine Lebergewebezelle ist.

3. *In-vitro*-Verfahren nach Anspruch 1 oder 2, wobei die verwendete Nucleinsäure Antisense-RNA (aRNA) oder ein rekombinantes DNA-Molekül umfaßt, das in eine aRNA transkribiert werden kann, wobei die aRNA mindestens teilweise zu mRNA komplementär ist, die für ein Hepatitis-Virus-Polymeraseprotein codiert, wobei die aRNA in der Lage ist, die virale Replikation in einer Zelle zu hemmen.

4. Verwendung von:
aRNA oder DNA nach Anspruch 3;
und/oder einem Vektor, der RNA oder ein DNA-Molekül nach Anspruch 3 umfaßt,
bei der Herstellung eines Mittels zür Hemmung oder Unterbrechung der Replikation von Hepatitis-Viren, wie ein Mittel zur Verhütung von Hepatitis.

## Revendications (Revendications pour l'(les) Etat(s) contractant(s) suivant(s): AT, BE, CH, LI, DE, DK, FR, GB, IT, LU, NL, SE)

1. Procédé in vitro d'inhibition de réplication du virus de l'hépatite dans une cellule, le procédé comprenant l'introduction dans la cellule d'un ARN antisens (ARNa) ou d'un ADN qui est transcrit en l'ARNa, l'ARNa étant au moins partiellement complémentaire d'une partie d'une région du gène de polymérase du génome du virus de l'hépatite.

2. Procédé in vitro suivant la revendication 1, dans lequel :
(a) le virus est le virus de l'hépatite B;
(b) l'ADN est régulé par un promoteur spécifique de la cellule, et/ou
(c) la cellule est une cellule de tissu hépatique.

3. ARN antisens (ARNa), ou une molécule d'ADN recombinée capable d'être transcrite en ARNa, l'ARNa étant au moins partiellement complémentaire à l'ARNm codant une protéine polymérase du virus de l'hépatite, où l'ARNa est capable d'inhiber la réplication du virus de l'hépatite dans une cellule.

4. ARNa ou ADN suivant la revendication 3, où la protéine polymérase du virus de l'hépatite provient du virus de l'hépatite B.

5. Vecteur comprenant une molécule d'ARNa ou d'ADN suivant la revendication 3 ou 4.

6. ARNa ou ADN suivant la revendication 3 ou 4, et/ou vecteur suivant la revendication 5 à utiliser en médecine.

7. Utilisation d'un ARNa ou ADN suivant la revendication 3 ou 4, et/ou vecteur suivant la revendication 5, dans la préparation d'un agent pour inhiber ou interrompre la réplication du virus de l'hépatite, en tant qu'agent de prévention de l'hépatite.

8. Composition adaptée à l'administration à un patient, comprenant :
un ARNa ou ADN suivant la revendication 3 ou 4,
et/ou vecteur suivant la revendication 5, et un support approprié.

## Revendications (Revendications pour l'(les) Etat(s) contractant(s) suivant(s): ES, GR)

1. Procédé in vitro d'inhibition de la réplication du virus de l'hépatite dans une cellule, le procédé comprenant l'introduction dans la cellule d'un ARN antisens (ARNa) ou d'un ADN qui est transcrit en l'ARNa, l'ARNa étant au moins partiellement complémentaire d'une partie d'une région du gène de polymérase du génome du virus de l'hépatite.

2. Procédé in vitro suivant la revendication 1, dans lequel :
(a) le virus est le virus de l'hépatite B;
(b) l'ADN est régulé par un promoteur spécifique de la cellule, et/ou
(c) la cellule est une cellule de tissu hépatique.

3. Procédé in vitro suivant la revendication 1 ou 2, dans lequel l'acide nucléique utilisé est un ARN antisens (ARNa), ou une molécule d'ADN recombinée capable d'être transcrite en un ARNa, l'ARNa étant au moins partiellement complémentaire à l'ARNm codant une protéine polymérase du virus de l'hépatite, où l'ARNa est capable d'inhiber la réplication virale dans une cellule.

4. Utilisation d'un ARNa ou ADN suivant la revendication 3, et/ou d'un vecteur comprenant une molécule d'ARN ou d'ADN suivant la revendication 3, dans la préparation d'un agent pour inhiber ou interrompre la réplication du virus de l'hépatite, en tant qu'agent de prévention de l'hépatite.
